# FASCICULE DE BREVET EUROPEEN

(11) **EP 2 042 076 B1**
(45) Date de publication et mention de la délivrance du brevet: **09.09.2015**
(21) Numéro de dépôt: 08163918.9
(22) Date de dépôt: 09.09.2008
(51) Int. Cl.: A61B 1/005

(54) **Structure orientable du type catheter ou endoscope**
Schwenkbare, bewegliche Struktur vom Typ Katheter oder Endoskop
Guidable structure such as a catheter or an endoscope

(30) Priorité: 26.09.2007 FR 0706726
(43) Date de publication de la demande: 01.04.2009
(73) Titulaire: Snecma, 75015 Paris (FR); Université Pierre et Marie Curie (Paris 6), 75005 Paris (FR); Centre National de la Recherche Scientifique, 75794 Paris Cedex 16 (FR)
(72) Inventeur: Bousquet, Sadia, 77550 Moissy Cramayel (FR); Szewcyk, Jérôme, 95510 Vienne en Arthies (FR)
(74) Mandataire: Ramey, Daniel

(56) Documents cités:
- EP-A- 0 279 316
- EP-A- 0 554 128
- EP-A2- 0 199 870
- WO-A-2004/084714
- US-A- 4 790 624
- US-B1- 6 240 231

## Description

La présente invention concerne une structure orientable du type cathéter ou endoscope destinée à l'exploration interne d'un système tridimensionnel, tel que par exemple une turbomachine.

Les cathéters ou endoscopes actuels se présentent sous la forme de longs tubes rigides ou élastiquement déformables ayant une extrémité orientable par rapport à leur axe longitudinal pour choisir un angle de vue particulier et faciliter la progression du cathéter ou de l'endoscope.

Afin de courber de manière adéquate une zone particulière du cathéter ou de l'endoscope, il est connu de disposer le long de sa structure des actionneurs se présentant sous la forme de fil en matériau à mémoire de forme, lesquels sont reliés à des moyens de chauffage par effet Joule. En effet, ces actionneurs voient leur longueur réduite sous l'effet d'une augmentation de la température, ce qui induit une modification de la courbure du cathéter ou de l'endoscope dans les zones où sont localisés les actionneurs. La commande des différents actionneurs répartis sur la longueur de l'endoscope ou cathéter permet de positionner son extrémité distale dans un espace tridimensionnel.

Cependant, ce type de dispositif présente plusieurs inconvénients. Le diamètre de l'extrémité distale est en général de l'ordre de 5 à 8 mm afin d'éviter des fléchissements sous l'effet de la gravité. Ce diamètre d'extrémité trop important rend impossible l'exploration de certaines zones critiques. Pour obtenir l'orientation angulaire voulue de l'extrémité du cathéter ou de l'endoscope, il est nécessaire de modifier des paramètres tels que la longueur et le diamètre des fils d'actionneurs ce qui est long et compliqué. Les dispositifs actuels souffrent également de limitations dues principalement au manque de mobilité et de manoeuvrabilité dans des espaces géométriquement complexes ou exigus. En effet, lors de la contraction d'un actionneur, le fléchissement provoqué localement du dispositif a un rayon de courbure sensiblement constant puisque la rigidité du dispositif est sensiblement constante sur toute sa longueur. Des lors, il n'est pas possible d'inspecter des cavités tridimensionnelles complexes ayant des passages de dimensions réduites et nécessitant différents changements d'orientation successifs.

Il en résulte que certaines zones d'une machine par exemple, restent inaccessibles lorsque l'on souhaite effectuer des contrôles non destructifs classiques telles que ceux réalisés par courants de Foucault ou par ultrasons, du fait de la complexité d'accessibilité, de cheminement et des faibles dimensions des passages à emprunter. Enfin, ces dispositifs connus ne sont pas pilotables automatiquement, ce qui complique d'autant la procédure d'exploration qui doit être effectuée manuellement.

Le document EP0199870, décrit une structure orientable pour l'exploration d'organes du corps humain, comprenant un corps longitudinal portant des éléments en matériau à mémoire de forme qui peuvent glisser par rapport au corps longitudinal lorsque l'un des éléments en matériau à mémoire de forme est chauffé par effet Joule pour être amené dans une configuration prédéterminée.

Le document EP0279316, décrit un mécanisme pour courber un corps longitudinal, comprenant un corps allongé dont une partie flexible présente une variation longitudinale de son module élastique. Une fibre optique s'étend à l'intérieur du corps longitudinal et est en contact avec un matériau à mémoire de forme agencé à l'intérieur de la partie flexible.

La présente invention a pour objet une structure du type décrit ci-dessus, qui évite les inconvénients précités de la technique antérieure de façon simple, efficace et économique, et permet d'accéder à des parties d'un système inaccessibles par les moyens connus.

Elle propose à cet effet, une structure orientable du type cathéter ou endoscope, destinée à l'observation ou au traitement d'un objet masqué, accessible par un passage étroit et/ou sinueux, comprenant un corps longitudinal élastiquement déformable comportant au moins un actionneur en matériau du type à mémoire de forme intégré longitudinalement au corps longitudinal et des moyens de chauffage par effet Joule permettant de contracter longitudinalement l'actionneur pour provoquer une flexion du corps longitudinal, caractérisée en ce que l'actionneur s'étend sur au moins une partie à rigidité variable du corps longitudinal.

Selon l'invention, la rigidité variable du corps longitudinal autorise ainsi un fléchissement faible dans les zones où la rigidité est importante et un fléchissement plus important dans les zones de rigidité plus faible ce qui permet d'obtenir un profil de courbure de la structure avec des rayons de courbure variables le long de la structure.

En adaptant la rigidité de la partie de la structure portant l'actionneur à la courbure désirée, on peut imposer l'orientation angulaire de l'extrémité distale de la structure, ce qui est plus simple et plus précis que dans la technique antérieure.

Selon une autre caractéristique de l'invention, la partie à rigidité variable de la structure comprend au moins une surépaisseur de matière, ce qui permet d'augmenter la rigidité dans cette zone par rapport aux zones sans surépaisseur, et de créer un profil à rayon de courbure variable lors de la contraction de l'actionneur.

La rigidité variable du corps longitudinal peut être déterminée pour réaliser, par contraction de l'actionneur, une modification et/ou une inversion de la courbure longitudinale ou transversale du cops longitudinal.

Le corps longitudinal et la surépaisseur peuvent être réalisés dans des matériaux semblables tels que par exemple un ou des polymères.

Dans un mode de réalisation, le corps longitudinal comprend au moins un tube dont le diamètre est par exemple de l'ordre de 2 à 6 mm, et l'actionneur s'étend longitudinalement sur une paroi interne ou externe du tube, sur au moins une partie de la longueur de celui-ci

Dans une autre variante de réalisation, le corps longitudinal comprend une lame à section transversale allongée et deux actionneurs parallèles sont incorporés à la lame et s'étendent le long d'une face longitudinale de la lame. La lame peut avoir une épaisseur d'environ 1 à 2 mm pour une largeur d'environ 1 cm et une longueur d'environ 5 à 10 cm.

Dans des systèmes tels que les turbomachines, les zones à explorer présentent une forte symétrie axiale. De ce fait, les passages que la structure doit emprunter ont souvent des sections de faible hauteur mais de grande largeur si bien que l'on peut envisager l'utilisation de structures à section transversale allongée, par exemple rectangulaire. L'utilisation d'une telle forme de structure permet de faire passer des outils supplémentaires tels que des pinces, fibres optiques, connexions diverses..., par l'intermédiaire de canaux s'étendant longitudinalement dans la structure. De plus, une structure à section transversale allongée résiste mieux à une sollicitation transversale appliquée dans le sens de la grande dimension de cette section.

Le nombre d'actionneurs peut également être augmenté du fait de la plus grande place disponible dans la section transversale, ce qui permet d'améliorer le contrôle de la courbure donnée à la structure.

L'actionneur utilisé pour fléchir l'endoscope peut être un fil en alliage de titane et de nickel, dont le diamètre est par exemple d'environ de 0,1 à 0,5 millimètre.

De façon plus générale, on peut utiliser pour ces actionneurs des matériaux ayant la propriété de se contracter et de diminuer de longueur quand ils sont chauffés, et notamment des matériaux tels que ceux appelés matériaux à mémoire de forme.

Selon une autre caractéristique de l'invention, la structure est du type télescopique et comprend plusieurs corps élastiquement déformables munis d'actionneurs et engagés les uns dans les autres.

Avantageusement, cette structure comprend à son extrémité distale des moyens élastiques exerçant une force longitudinale de poussée et reliés à une tête pourvue de moyens de contrôle non destructif.

Dans cette configuration, l'extrémité distale de la structure n'est pas liée de manière rigide au corps élastique, mais lui est rattachée par des moyens de poussée qui permettent d'assurer un contact permanent entre la surface de la pièce à étudier et les moyens de contrôle non destructif portés par la tête de la structure.

Les moyens de poussée peuvent être des ressorts dont certains au moins sont en matériau contractile à mémoire de forme et sont reliés à des moyens de chauffage par effet Joule.

L'utilisation de ressorts en matériau contractile permet de faire varier leur raideur pour régler au mieux la pression exercée sur la surface de la pièce examinée.

Les moyens de contrôle non destructif peuvent être des sondes à courants de Foucault ou des sondes à ultrasons, par exemple.

Une dimension transversale de la structure peut varier entre environ 8 mm à son extrémité proximale et environ 1 mm à son extrémité distale. Par ailleurs, cette structure peut comprendre des moyens d'accrochage ou d'appui à des éléments fixes environnants.

En effet, la progression de la structure à l'intérieur d'un système, réalisée par emboîtements successifs de plusieurs structures individuelles orientables peut conduire à un déplacement de son extrémité distale par fléchissement de la structure. Les moyens d'accrochage ou d'appui permettent alors de se positionner de façon précise sur des éléments fixes intermédiaires afin de limiter l'effet de la gravité sur la structure et d'améliorer le contrôle du positionnement et de l'orientation de son extrémité. Cela permet aussi de réaliser des structures du type cathéter ou endoscope plus longues et plus fines.

Les moyens d'alimentation électrique des actionneurs, provoquant leur chauffage, peuvent être pilotés automatiquement, ce qui permet dans le cas d'un système de géométrie connue, d'effectuer le déplacement de la structure à l'intérieur du système exploré de manière automatique et ainsi de s'affranchir des nombreuses difficultés inhérentes au contrôle continu des actionneurs à base de matériau contractile.

L'invention sera mieux comprise et d'autres détails, avantages et caractéristiques de l'invention apparaîtront à la lecture de la description suivante faite à titre d'exemple non limitatif, en référence aux dessins annexés dans lesquels :
- la figure 1 est une vue schématique en coupe axiale d'une structure tubulaire orientable comprenant un actionneur en matériau contractile selon la technique antérieure ;
- la figure 2 est une vue schématique en coupe axiale de la structure selon la figure 1, courbée par chauffage du matériau contractile ;
- la figure 3 est une vue schématique en coupe axiale d'une structure tubulaire orientable à rigidité variable selon l'invention ;
- la figure 4 est une vue schématique de la structure tubulaire de la figure 3, courbée par chauffage du matériau contractile ;
- la figure 5 est une vue schématique en perspective d'une structure à section transversale allongée et à rigidité variable ;
- la figure 6 est une vue de la structure de la figure 5, courbée par chauffage du matériau contractile ;
- les figures 7 et 8 sont des vues schématiques en coupe axiale d'une structure courbée en forme de « S » et d'une structure en forme de crochet selon l'invention ;
- les figures 9 et 10 sont des vues schématiques en perspective de structures comprenant des ressorts de poussée à leur extrémité distale ;
- la figure 11 est une vue schématique en coupe axiale d'une structure orientable à rigidité variable dont l'extrémité distale comprend des ressorts de poussée ;
- la figure 12 est une vue schématique en coupe axiale d'une partie d'une turbomachine destinée à être explorée à l'aide d'une structure orientable selon l'invention.

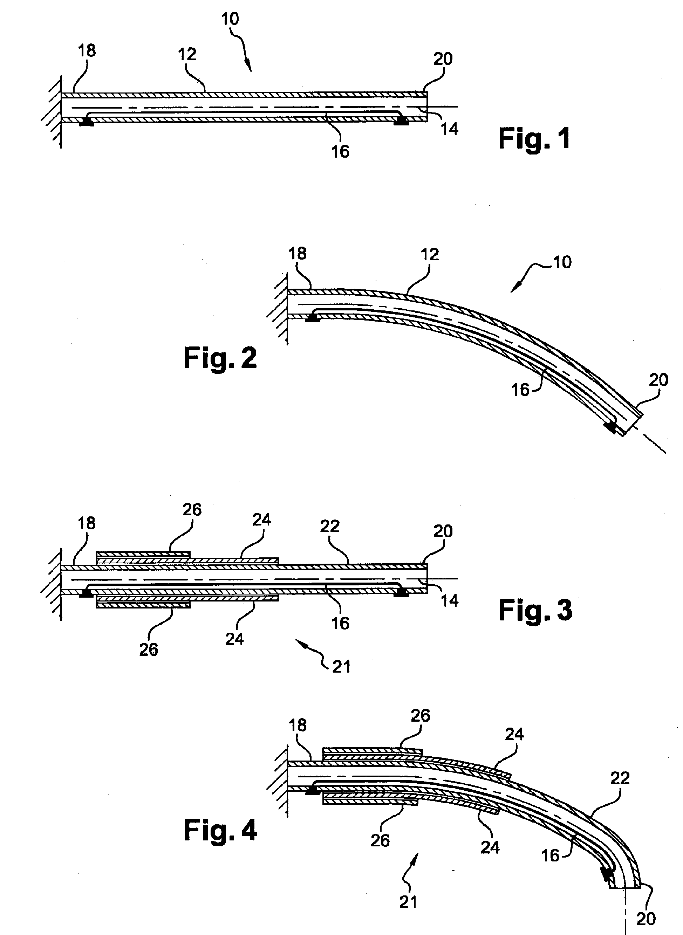

On se réfère d'abord à la figure 1 qui est une vue schématique d'une structure orientable 10 du type cathéter ou endoscope selon la technique antérieure, comprenant un corps flexible tubulaire 12 d'axe longitudinal 14 et comportant un actionneur 16 en matériau contractile, par exemple du type à mémoire de forme (ci-après dénommé AMF) disposé le long de la structure tubulaire et à l'intérieur de celle-ci. Cet actionneur 16 se présente sous la forme d'un fil relié à des moyens d'alimentation électrique (non représentés). La structure orientable 10 comprend une extrémité proximale 18 fixe et une extrémité distale 20 orientable.

Le chauffage par effet Joule du fil en AMF, induit un réarrangement des atomes constituant le fil (lorsque la température d'activation est atteinte), conduisant à une contraction (avec un temps de réponse inférieur à une seconde) et donc à une diminution de sa longueur. Le fil 16 fixé à une paroi du tube 12 induit un fléchissement de son extrémité distale 20 dans une direction perpendiculaire à l'axe 14 (figure 2). La structure 10 ainsi fléchie adopte un profil en arc de cercle, de rayon de courbure sensiblement constant dans la mesure où la rigidité le long du tube 12 est sensiblement constante. Un tel cathéter ne permet une orientation angulaire de son extrémité distale 20 que de quelques degrés autour de l'axe longitudinal 14 s'il faut limiter le déplacement latéral relatif entre les deux extrémités 18, 20 du cathéter. Une modification de l'orientation angulaire plus importante conduit à une augmentation du déplacement latéral de l'extrémité distale 20 du cathéter. Une telle structure ne peut donc être utilisée que dans des systèmes où les passages sont étroits et avec des modifications angulaires faibles ou bien dans des systèmes ayant des passages suffisamment grands pour autoriser un important débattement latéral, ce qui représente une forte limitation à l'exploration interne de systèmes très complexes et exigus tels qu'ils existent en aéronautique, par exemple.

L'arrêt du chauffage par effet Joule du fil conduit à un refroidissement du fil 16 et à un retour à l'état initial longitudinal de la structure 10 par effet élastique.

Selon l'invention, et comme représenté en figure 3, la structure 21 comprend un corps flexible 22 comportant au moins une partie à rigidité variable sur laquelle s'étend le fil 16 en AMF. Cette variation de rigidité est obtenue en disposant ou en formant des surépaisseurs de matière sur la surface extérieure du tube 22. Dans l'exemple représenté, une première surépaisseur de matière 24 est disposée autour du tube 22 puis une deuxième surépaisseur 26 de dimension axiale plus petite que la première est disposée autour de la première surépaisseur 24. Ainsi le corps flexible présente de son extrémité proximale 18 vers son extrémité distale 20 une rigidité décroissante.

Lors de la contraction du fil 16 par chauffage par effet Joule, le tube est fléchi selon un rayon de courbure variable (figure 4). En effet, le tube se courbe faiblement dans sa partie présentant une importante rigidité et se courbe de manière plus importante dans la zone de rigidité plus faible. On constate également que le débattement latéral est plus faible et donc l'encombrement réduit par rapport à la technique antérieure pour une même orientation angulaire de l'extrémité distale 20.

Ainsi, il est possible d'obtenir des changements d'orientation de l'extrémité distale atteignant 90° par rapport à l'axe longitudinal 14 pour un faible débattement latéral. Ceci est réalisable dans la mesure où le tube 22 ne présente pas un diamètre trop important au regard des dimensions du fil 16 en AMF.

Typiquement, le diamètre du fil 16 est de l'ordre de 0,5 à 0,1 millimètres et sa contraction longitudinale, obtenue par chauffage par effet Joule (produit par une puissance électrique d'environ 0,5 Watt), est de l'ordre de 5 à 6 % de sa longueur. Le tube 22 a un diamètre de l'ordre de 2 à 6 millimètres et est en matériau polymère, à l'image des cathéters médicaux existants. Le réglage de l'intensité du courant électrique passant dans les actionneurs permet aussi de régler la contraction et donc la courbure du corps 22.

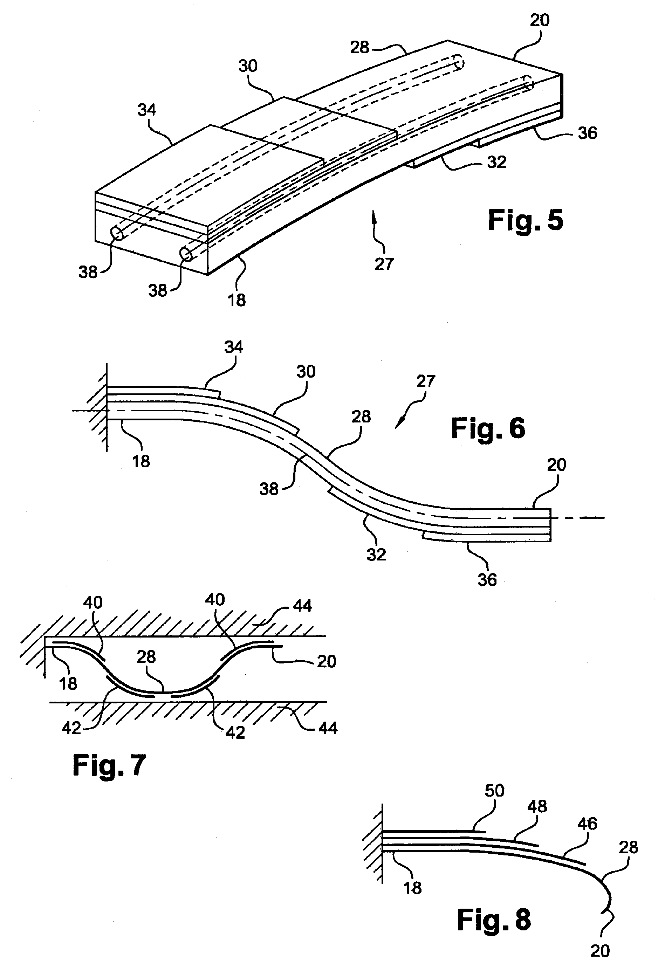

Les passages empruntés par les endoscopes ou cathéters pour l'inspection de machines ont souvent la forme de fentes étroites, notamment dans les systèmes à symétrie axiale comme dans les turbomachines. Il n'est donc pas nécessaire que le cathéter soit tubulaire et il peut adopter une autre forme. L'invention peut donc également être appliquée à un corps flexible longitudinal 28 de section transversale allongée tel que celui représenté en figure 5, où le corps 28 se présente sous la forme d'une lame de section sensiblement rectangulaire.

De manière similaire au mode de réalisation précédent, le cathéter 27 comprend un corps flexible 28 comportant des couches de matière disposées en surépaisseur afin de modifier la rigidité de la lame dans la direction longitudinale. Ces couches supplémentaires de matière sont formées de lamelles de section également rectangulaire. La structure comprend ici quatre couches de matière ou lamelles ajoutées, de largeur identique à celle de la lame 28. Une première lamelle 30 est positionnée sur une face du corps 28 tandis qu'une autre lamelle 32 de même longueur que la première est positionnée sur la face opposée, à l'autre extrémité du corps longitudinal 28. Deux autres lamelles 34, 36 de même longueur sont disposées respectivement sur chacune des deux premières lamelles 30, 32 et sont de longueurs inférieures aux lamelles 30 et 32.

Deux fils 38 parallèles contractiles ou en AMF sont incorporés à la lame 28 et s'étendent parallèlement à ses faces longitudinales dans son plan médian.

Par chauffage par effet Joule des fils 38, on obtient une structure courbée en forme de « S » (figure 6) puisque de l'extrémité proximale 18 jusqu'à l'extrémité distale 20 du corps longitudinal, la rigidité est décroissante puis constante sur une face de la lame 28, et inversement sur l'autre face. Cette structure peut être particulièrement utile lorsqu'il s'agit de réaliser un décalage de l'extrémité distale 20, perpendiculairement à l'axe longitudinal, sans modifier son orientation angulaire, ou plus généralement, lorsqu'il faut non seulement modifier la courbure du corps longitudinal, mais aussi l'inverser dans une certaine zone.

La structure de la figure 7 représente une forme en « cuvette ». Une telle forme peut être obtenue en ajoutant une lamelle 40 en surépaisseur du corps flexible 28 à chacune de ses extrémités, ces deux lamelles étant disposées sur la même face tandis que la face opposée comprend deux lamelles 42 alignées le long du corps 28 et disposées entre les lamelles d'extrémité 40. Ce type de structure peut être utilisé afin de stabiliser un endoscope ou un cathéter par appui sur des éléments fixes environnants 44, ce qui limite les effets de la gravité sur l'ensemble de la structure et permet d'utiliser des cathéters plus longs et plus fins que s'ils étaient constitués de structures uniquement autoporteuses. Les moyens d'accrochage ou d'appui peuvent également se révéler particulièrement utiles lors d'une opération de contrôle non destructif, décrite ultérieurement, qui nécessite une position stable de l'extrémité distale 20 du cathéter.

La structure en forme de « crochet » de la figure 8 peut être obtenue en utilisant trois couches de matière 46, 48, 50 de longueurs différentes empilées les unes sur les autres sur une même face de la lame 28. Ainsi, l'extrémité proximale 18 du corps longitudinal 28 ne subit qu'un faible fléchissement tandis que la majorité du fléchissement est concentrée à son extrémité distale 20, ce qui permet d'obtenir un déplacement angulaire de l'extrémité distale 20 qui peut être supérieur à 90° tout en conservant un débattement latéral faible.

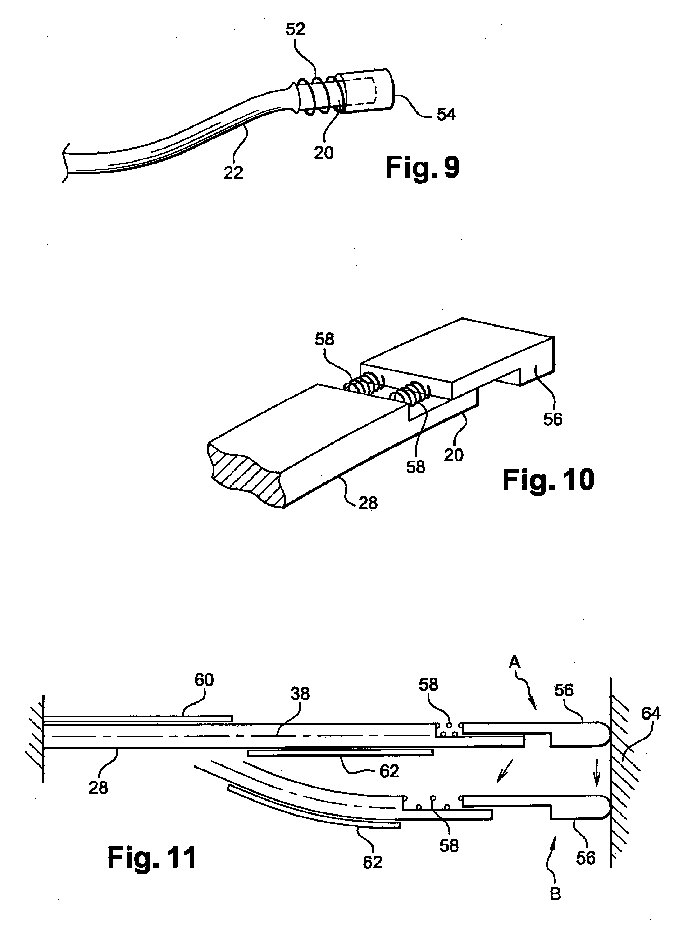

Comme représenté aux figures 9 et 10, les cathéters ou endoscopes peuvent comprendre des moyens élastiques exerçant une force longitudinale de poussée sur une tête pourvue de moyens de contrôle non destructif. Ces moyens élastiques comprennent par exemple des ressorts hélicoïdaux.

Dans le cas d'un endoscope ou cathéter tubulaire (figure 9), un ressort hélicoïdal 52 est enroulé autour de l'extrémité distale 20 du corps flexible 22 et est en appui sur un rebord du corps longitudinal 22, l'autre extrémité du ressort 52 étant en appui sur le fond d'un élément en forme de capuchon 54 entourant le ressort 52.

Dans le cas où le corps flexible 28 (figure 10) est à section transversale allongée, par exemple rectangulaire, son extrémité distale 20 peut comprendre un décrochement rectangulaire sur lequel est guidée une tête 56 de forme complémentaire. La tête 56 est reliée au corps longitudinal 28 par deux ressorts 58 disposés en parallèle.

Lorsque le cathéter est amené à proximité de la surface d'une pièce d'intérêt, les ressorts 52, 58 permettent de maintenir la tête 54, 56 en contact avec la surface pendant le temps nécessaire à l'examen de la pièce par les moyens de contrôle non destructif.

Les ressorts 52, 58 peuvent être réalisés en matériau contractile ou à mémoire de forme dans le cas où le contrôle de la raideur du ressort ou bien de la force appliquée s'avère intéressant. De tels ressorts peuvent également permettre de contrôler avec une grande précision la distance entre l'extrémité de la tête et la surface.

La figure 11 représente deux positions d'un cathéter ou endoscope selon l'invention, l'une (A) correspondant à l'état initial avant déformation élastique et l'autre (B) correspondant à l'état après déformation élastique. Le cathéter est du type à section transversale allongée et comprend deux couches de matière 60, 62 disposées sur des faces opposées aux extrémités du corps flexible 28 et deux fils 38 en AMF comme décrit pour la figure 5 ainsi qu'un ou des ressorts 58 reliant la tête 56 au corps élastique 28, la tête 56 étant en contact avec une surface d'une pièce d'intérêt 64.

Lors du chauffage par effet Joule, le cathéter adopte une forme en « S » selon le principe décrit précédemment, ce qui permet un décalage de l'extrémité distale perpendiculairement à l'axe longitudinal sans modifier son orientation. Le ou les ressorts 58 ont pour fonction de maintenir la tête en contact permanent avec la surface de la pièce 64.

Grâce à ce dispositif et en plaçant des moyens de contrôle non destructif au niveau de la tête 56, il est possible de réaliser une exploration par balayage de l'intérieur de la pièce 64, en effectuant un déplacement rectiligne de la tête 56 par actionnement répétitif des fils 38 en AMF.

Des moyens de contrôle non destructif tels que des sondes à courants de Foucault ou des sondes à ultrasons peuvent s'avérer particulièrement utiles pour détecter des criques de surfaces.

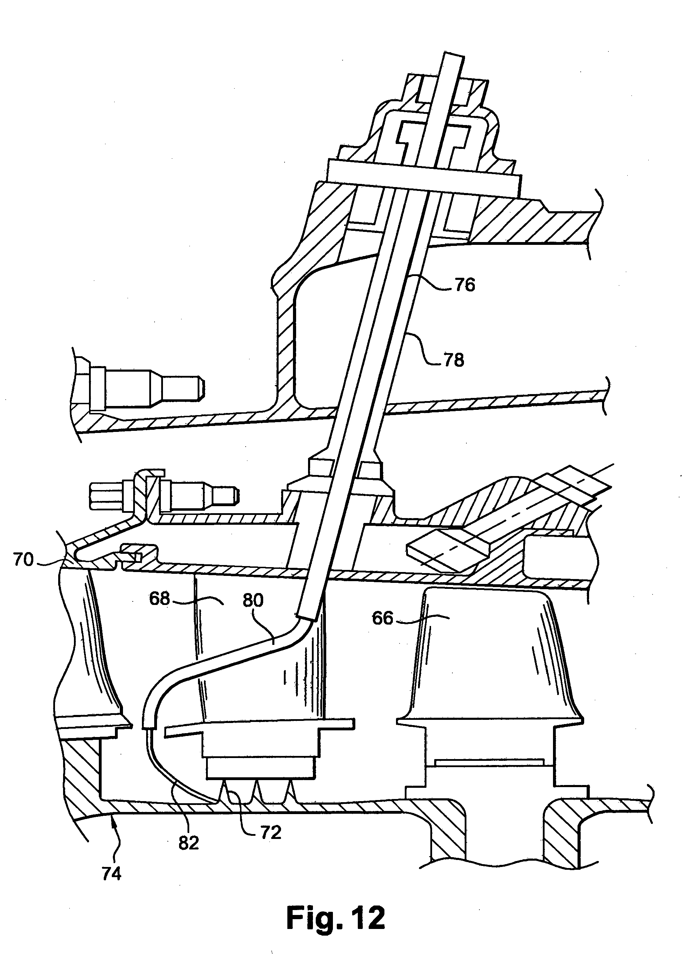

La figure 12 représente un étage d'une turbomachine comportant une alternance d'aubes mobiles 66 et d'aubes fixes 68 entourées par un carter externe 70. Les aubes fixes 68 ont leur extrémité interne radialement alignée avec des léchettes 72 montées sur une partie du rotor 74. Ces léchettes 72 permettent d'éviter toute circulation d'air entre une aube fixe 68 et le rotor 74. Il est donc important de pouvoir contrôler l'état d'usure de ces pièces pour éviter toute diminution des performances de la turbomachine.

Les cathéters ou endoscopes utilisés à cette fin sont du type télescopique c'est-à-dire qu'ils sont constitués d'une pluralité de corps élastiquement déformables munis d'actionneurs contractiles et engagés les uns dans les autres.

Un cathéter selon l'invention permet de contrôler de manière simple et rapide ces léchettes 72. Pour cela un premier corps rigide tubulaire 76 est introduit dans un orifice endoscopique 78 débouchant entre deux aubes fixes adjacentes 68 et un deuxième corps flexible 80 de forme en « S » est introduit à l'intérieur du premier corps. Les moyens de chauffage des actionneurs permettent une mise en forme de ce cathéter 80 dans l'espace entre deux aubes fixes 68. Enfin, un troisième corps flexible 82 possédant un seul sens de courbure est introduit à l'intérieur des deux premiers cathéters 78, 80 et est commandé afin que sa tête puisse être placée en contact avec une léchette 72. Le rotor de la turbomachine est ensuite mis en rotation ce qui permet grâce aux moyens de contrôle non destructif placés au niveau de la tête du cathéter 82 d'examiner l'état de surface de la pièce sur 360°. La tête peut être reliée à un ressort comme décrit précédemment afin d'assurer un contact permanent.

La longueur totale du cathéter est relativement importante, par exemple d'environ 60 centimètres, et peut induire des erreurs de positionnement de son extrémité distale. On peut utiliser les aubes fixes 68 comme points d'appui et d'accrochage du cathéter intermédiaire 80 à l'aide d'éléments tels que des pinces ou treillis déployables pour stabiliser l'ensemble du cathéter télescopique.

La configuration à rigidité variable permet de manoeuvrer le cathéter jusqu'à des zones difficilement accessibles qui requièrent son cheminement dans des passages de faibles dimensions.

Les cathéters à section transversale allongée peuvent être pleins comme représentés sur les dessins ou bien creux. Dans le cas de cathéters télescopiques pleins, on peut prévoir des moyens de guidage tels que des rails, situés sur le long des corps longitudinaux.

Dans les différentes variantes de réalisation décrites, on peut avoir un nombre variable de fils contractile ou en matériau à mémoire de forme et un nombre variable de couches en surépaisseur afin d'obtenir des profils de courbure voulue.

L'invention n'est pas non plus limitée au type d'actionneurs AMF utilisés dans les modes de réalisation représentés aux dessins, à savoir des fils AMF simple effet, c'est-à-dire dont le mode d'action est limité à un sens. On peut aussi utiliser d'autres actionneurs comme des lames AMF, possédant éventuellement deux positions mémorisées afin d'obtenir un retour à l'état initial plus rapide de la structure orientable. Il est également envisageable afin de produire un retour à l'état initial plus rapide de disposer des fils en AMF le long du corps élastique et en position antagoniste, et de les actionner successivement.

Si l'invention décrite précédemment est particulièrement utile dans le domaine de l'exploration tridimensionnelle de dispositifs industriels complexes, elle peut également être utilisée dans d'autres domaines et en particulier dans le domaine biomédical où la manoeuvrabilité des cathéters est un point clef d'une exploration anatomique et fonctionnelle réussie.

L'invention concerne également un cathéter ou endoscope comprenant des moyens automatiques de commande reliés à chacun des actionneurs contractiles. Ceci est particulièrement intéressant dans le cas de systèmes de géométrie parfaitement connue. En utilisant les plans du système, il est possible de définir avec précision le chemin à suivre et la forme à donner à la structure, et de programmer la commande des actionneurs à partir de l'introduction de la structure dans un orifice endoscopique.

Du fait de la simplicité de sa mise en oeuvre et du faible coût, l'invention autorise la fabrication de cathéters dédiés à l'exploration de systèmes d'un type donné et au contrôle non destructif d'un seul type de pièces.

On peut ainsi optimiser la fabrication d'une structure orientable selon l'invention en fonction de la zone et du type de contrôle non destructif souhaité, ce qui permet un gain important de performances pour l'utilisateur.

Le matériau contractile peut être par exemple un alliage de titane et de nickel. Le corps élastique ainsi que les couches de matière peuvent être réalisés en acier à ressort ou en un polymère tel que le polyethyletherketone, l'époxy, le polyéthylène ou le polyuréthane suivant la rigidité souhaitée.

L'invention n'est pas limitée aux cathéters de section rectangulaire ou circulaire et s'applique également à des cathéters de section transversale quelconque, par exemple ovale, carrée, triangulaire, etc.

## Revendications

1. Structure orientable du type cathéter ou endoscope, destinée à l'observation ou au traitement d'un objet masqué, accessible par un passage étroit et/ou sinueux, comprenant un corps longitudinal élastiquement déformable (22, 28) comportant au moins un actionneur (16, 38) en matériau du type à mémoire de forme intégré longitudinalement au corps longitudinal (22, 28) et des moyens de chauffage par effet Joule permettant de contracter longitudinalement l'actionneur (16, 38) pour provoquer une flexion du corps longitudinal (22, 28), **caractérisée en ce que** l'actionneur (16, 38) s'étend sur au moins une partie à rigidité variable du corps longitudinal (22, 28) et **en ce que** l'extrémité distale du corps longitudinal (20) comprend des moyens élastiques (52, 58) exerçant une force longitudinale de poussée et reliés à une tête (54, 56) pourvue de moyens de contrôle non destructif.

2. Structure selon la revendication 1, **caractérisée en ce que** la partie à rigidité variable comprend au moins une surépaisseur de matière.

3. Structure selon la revendication 1 ou 2, **caractérisée en ce que** la rigidité variable du corps longitudinal (22, 28) est déterminée pour réaliser, par contraction de l'actionneur (16, 38), une modification et/ou une inversion de la courbure longitudinale ou transversale du corps longitudinal (22, 28).

4. Structure selon la revendication 1, 2 ou 3, **caractérisée en ce que** le corps longitudinal (22, 28) comprend au moins un tube et **en ce que** l'actionneur (16) s'étend longitudinalement sur une paroi interne ou externe du tube.

5. Structure selon la revendication 1, 2 ou 3, **caractérisée en ce que** le corps longitudinal comprend une lame à section transversale allongée.

6. Structure selon la revendication 5, **caractérisée en ce que** deux actionneurs parallèles (38) sont incorporés à la lame et s'étendent le long d'une face longitudinale de la lame.

7. Structure selon l'une des revendications précédentes, **caractérisée en ce que** l'actionneur (16, 38) est un fil.

8. Structure selon l'une des revendications 2 à 7, **caractérisée en ce que** la partie à rigidité variable et la surépaisseur de matière sont en polymère.

9. Structure selon l'une des revendications précédentes, **caractérisée en ce que** l'actionneur est en alliage composé de titane et de nickel.

10. Structure selon l'une des revendications précédentes, **caractérisée en ce qu'**elle est du type télescopique et comprend plusieurs corps élastiquement déformables (22, 28) munis d'actionneurs (16, 38) et engagés les uns dans les autres.

11. Structure selon l'une des revendications précédentes, **caractérisée en ce que** les moyens de poussée comprennent des ressorts (52, 58) en matériau du type à mémoire de forme et qui sont reliés à des moyens de chauffage par effet Joule.

12. Structure selon l'une des revendications précédentes, **caractérisée en ce que** les moyens de contrôle non destructif sont des sondes à courants de Foucault ou des sondes à ultrasons.

13. Structure selon l'une des revendications précédentes, **caractérisée en ce qu'**elle comprend des moyens d'accrochage ou d'appui sur des éléments fixes environnants.

14. Structure selon l'une des revendications précédentes, caractérisée en ce les moyens de chauffage sont reliés à des moyens de commande automatique, par exemple programmée.

## Patentansprüche

1. Schwenkbare, bewegliche Struktur vom Typ Katheter oder Endoskop, die zur Beobachtung bzw. Behandlung eines verdeckten Objekts bestimmt ist, das über einen engen und/oder gewundenen Durchgang zugänglich ist, enthaltend einen elastisch verformbaren longitudinalen Körper (22, 28), der zumindest ein Betätigungsglied (16, 38) aus einem formspeichernden Material enthält, das longitudinal im longitudinalen Körper (22, 28) integriert ist, sowie Heizeinrichtungen zum Erwärmen durch Joule-Effekt, die gestatten, das Betätigungsglied (16, 38) longitudinal zusammenzuziehen, um eine Biegung des longitudinalen Körpers (22, 28) hervorzurufen, **dadurch gekennzeichnet, dass** das Betätigungsglied (16, 38) sich zumindest über einen Teil veränderlicher Steifigkeit des longitudinalen Körpers (22, 28) erstreckt und dass das distale Ende des longitudinalen Körpers (20) Federmittel (52, 58) aufweist, die eine longitudinale Schubkraft ausüben und mit einem Kopf (54, 56) verbunden sind, der mit Prüfeinrichtungen zur zerstörungsfreien Prüfung versehen ist.

2. Struktur nach Anspruch 1, **dadurch gekennzeichnet, dass** der Teil mit veränderlicher Steifigkeit zumindest eine Material-Überdicke aufweist.

3. Struktur nach Anspruch 1 oder 2, **dadurch gekennzeichnet, dass** die veränderliche Steifigkeit des longitudinalen Körpers (22, 28) so bestimmt ist, dass sie durch Zusammenziehen des Betätigungsglieds (16, 38) eine Änderung und/oder Umkehrung der längs oder quer verlaufenden Krümmung des longitudinalen Körpers (22, 28) bewirkt.

4. Struktur nach Anspruch 1, 2 oder 3, **dadurch gekennzeichnet, dass** der longitudinale Körper (22, 28) zumindest eine Röhre aufweist und dass das Betätigungsglied (16) sich longitudinal über eine Innenwand oder Außenwand der Röhre erstreckt.

5. Struktur nach Anspruch 1, 2 oder 3, **dadurch gekennzeichnet, dass** der longitudinale Körper einen Steg mit langgestrecktem Querschnitt aufweist.

6. Struktur nach Anspruch 5, **dadurch gekennzeichnet, dass** zwei parallel verlaufende Betätigungsglieder (38) in dem Steg eingegliedert sind und sich entlang einer Längsseite des Stegs erstrecken.

7. Struktur nach einem der vorangehenden Ansprüche, **dadurch gekennzeichnet, dass** das Betätigungsglied (16, 38) ein Draht ist.

8. Struktur nach einem der Ansprüche 2 bis 7, **dadurch gekennzeichnet, dass** der Teil mit veränderlicher Steifigkeit und die Material-Überdicke aus Polymer bestehen.

9. Struktur nach einem der vorangehenden Ansprüche, **dadurch gekennzeichnet, dass** das Betätigungsglied aus einer aus Titan und Nickel zusammengesetzten Legierung besteht.

10. Struktur nach einem der vorangehenden Ansprüche, **dadurch gekennzeichnet, dass** sie teleskopartig ausgeführt ist und mehrere elastisch verformbare Körper (22, 28) aufweist, die mit Betätigungsgliedern (16, 38) versehen sind und ineinander eingreifen.

11. Struktur nach einem der vorangehenden Ansprüche, **dadurch gekennzeichnet, dass** die Schubeinrichtungen Federn (52, 58) aus formspeicherndem Material aufweisen, die mit den Heizeinrichtungen zum Erwärmen durch Joule-Effekt verbunden sind.

12. Struktur nach einem der vorangehenden Ansprüche, **dadurch gekennzeichnet, dass** die Prüfeinrichtungen zur zerstörungsfreien Prüfung Wirbelstromsonden oder Ultraschallsonden sind.

13. Struktur nach einem der vorangehenden Ansprüche, **dadurch gekennzeichnet, dass** sie Verhakungs- oder Abstützeinrichtungen zum Verhaken bzw. Abstützen auf festen, umliegenden Elementen aufweist.

14. Struktur nach einem der vorangehenden Ansprüche, **dadurch gekennzeichnet, dass** die Heizeinrichtungen mit Steuerungseinrichtungen zur automatischen, beispielsweise programmierten Steuerung verbunden sind.

## Claims

1. A steerable structure of catheter or endoscope type for observing or treating a masked object that is accessible via a passage that is narrow and/or sinuous, the structure comprising an elastically deformable longitudinal body (22, 28) having at least one actuator(16, 38) of shape memory type material incorporated longitudinally in the longitudinal body (22, 28) together with Joule-effect heater means enabling the actuator (16, 38) to be contracted longitudinally so as to cause the longitudinal body (22, 28) to bend, **characterized in that** the actuator (16, 38) extends over at least one portion of the longitudinal body (22, 28) that presents varying stiffness, and **in that** the distal end of the longitudinal body (20) includes resilient means (52, 58) exerting a longitudinal thrust force and connected to a head (54, 56) that is provided with non-destructive inspection means.

2. A structure according to claim 1, **characterized in that** the portion of varying stiffness comprises at least one extra thickness of material.

3. A structure according to claim 1 or 2, **characterized in that** the varying stiffness of the longitudinal body (22, 28) is designed so that contraction of the actuator (16, 38) modifies and/or inverts the longitudinal or transverse curvature of the longitudinal body (22, 28).

4. A structure according to claim 1, 2 or 3, **characterized in that** the longitudinal body (22, 28) comprises at least one tube, and **in that** the actuator (16) extends longitudinally over an inside wall or an outside wall of the tube.

5. A structure according to claim 1, 2 or 3, **characterized in that** the longitudinal body comprises a blade of elongate cross-section.

6. A structure according to claim 5, **characterized in that** two parallel actuators (38) are incorporated in the blade and extend along a longitudinal face of the blade.

7. A structure according to any of the preceding claims, **characterized in that** the actuator is a wire.

8. A structure according to one of the claims 2 to 7, **characterized in that** the portion of varying stiffness and the extra thickness of material are made of polymer.

9. A structure according to any of the preceding claims, **characterized in that** the actuator is made of an alloy of titanium and nickel.

10. A structure according to any of the preceding claims of, **characterized in that** it is of the telescopic type and comprises a plurality of elastically deformable bodies (22, 28) provided with actuators (16, 38) and engaged one in another.

11. A structure according to any of the preceding claims, **characterized in that** the thrust means are springs (52, 58), the springs being made of shape memory type material and are connected to Joule-effect heater means.

12. A structure according to any of the preceding claims, **characterized in that** the non-destructive inspection means are Foucault current probes or ultrasound probes.

13. A structure according any of the preceding claims, **characterized in that** it comprises means for hanging on or bearing against surrounding stationary elements.

14. A structure according to any of the preceding claims, **characterized in that** the heater means are connected to automatic control means, e.g. programmed means.
